# FASCICULE DE BREVET EUROPEEN

(11) **EP 1 206 242 B1**
(45) Date de publication et mention de la délivrance du brevet: **28.12.2005**
(21) Numéro de dépôt: 99939501.5
(22) Date de dépôt: 26.08.1999
(51) Int. Cl.: A61K 7/48

(54) **UTILISATION D'EXTRAITS BACTERIENS DE LA FAMILLE DES PSEUDOMONADACEES COMME AGENTS COSMETIQUES**
VERWENDUNG VON EXTRAKTEN VON PSEUDOMONACEAE-BAKTERIEN ALS KOSMETISCHE WIRKSTOFFE
USE OF BACTERIAL EXTRACTS OF THE PSEUDOMONADACEAE FAMILY AS COSMETIC AGENTS

(43) Date de publication de la demande: 22.05.2002
(73) Titulaire: L'OREAL, 75008 Paris (FR)
(72) Inventeur: MARTIN, Richard, F-37210 Rochecorbon (FR); HILAIRE, Pascal, F-37210 Vouvray (FR); PINEAU, Nathalie Résidence des jardins du Clain, F-86000 Poitiers (FR); BRETON, Lionel, F-78000 Versailles (FR)
(74) Mandataire: Le Coupanec, Pascale A.M.P.
(86) Numéro de dépôt international: PCT/FR1999/002043
(87) Numéro de publication internationale: WO 2001/013878

(56) Documents cités:
- EP-A- 0 404 660
- EP-A- 0 404 661
- EP-A- 0 631 773
- DE-A- 19 628 454
- DE-A- 19 824 073
- FR-A- 2 775 186
- US-A- 5 856 451
- STN, Serveur de Bases de Données, Karlsruhe, DE, Fichier Chemical Abstracts, Vol 124, AN=241783 résumé XP002138245 & JP 08 003018 A (OOTOMO)
- STN, Serveur de Bases de Données, Karslruhe, DE, Fichier Chemical Abstracts, Vol 125, AN=606985 résumé XP002138246 & JP 08 208427 A (NARISU COSMETIC CO)

## Description

L'invention a pour objet l'utilisation d'extraits de bactéries de la famille de Pseudomonadacées comme agents cosmétiques permettant notamment de lutter contre le vieillissement cutané chez l'homme.

On sait que le vieillissement cutané se manifeste en premier lieu par une diminution du nombre et une fragmentation des fibres élastiques du derme. L'élastine devient plus sensible à la lyse par l'élastase, et l'altération de l'élastine conduit à une désorganisation des fibres élastiques. Ces phénomènes se traduisent par une perte de l'élasticité de la peau et par la formation de rides.

Une autre manifestation du vieillissement cutané est la sécheresse de la peau, qui devient rêche, avec perte de la flexibilité de l'épiderme et tendance à la desquamation. Dans le tissu conjonctif de la peau de sujets jeunes, la teneur élevée en hyaluronates, fortement hydrophiles, favorise l'hydratation du derme, qui est un élément essentiel de la tonicité de la peau. Au cours du vieillissement, la teneur en hyaluronates, et donc la teneur en eau du derme, diminue fortement, avec pour conséquences fâcheuses la flaccidité de la peau et une moindre diffusion de l'eau dermique vers l'épiderme, qui se dessèche. En outre, la diminution de la teneur en eau du derme a notamment pour conséquence de freiner la circulation des métabolites, des ions et de l'oxygène, et donc de ralentir le métabolisme des cellules du derme et de l'épiderme. La diminution de la teneur en hyaluronates est liée à l'activité d'une enzyme, la hyaluronidase, qui clive les liaisons glucosidiques des hyaluronates. C'est pourquoi cette enzyme joue un rôle très important dans le vieillissement cutané.

En outre, le dessèchement de l'épiderme diminue les échanges gazeux avec l'atmosphère ambiante à la surface de la peau. Ce phénomène d'échange gazeux, appelé respiration cutanée, diminue avec l'âge.

Par ailleurs, on sait que l'exposition au soleil est susceptible de provoquer une réaction inflammatoire dans le tissu cutané, et qu'après des expositions au soleil, en particulier aux UVA, répétées et prolongées, la peau devient à terme desséchée, excessivement ridée, et dépourvue de souplesse : ce vieillissement prématuré de la peau est appelé "photovieillissement".

Il est donc souhaitable de trouver de nouveaux moyens permettant notamment de protéger la peau contre le vieillissement accéléré ou prématuré de la peau, et permettant de mieux protéger la peau contre les dommages causés par l'exposition au soleil, y compris contre le photovieillissement cutané.

On a maintenant découvert que des extraits de bactéries de la famille des Pseudomonadacées, et en particulier de bactéries du genre *Pseudomonas,* lorsqu'ils sont appliqués sur la peau, sont capables notamment d'améliorer l'hydratation de la peau et de protéger la peau contre certaines conséquences néfastes des réactions inflammatoires consécutives à l'exposition aux ultraviolets. Plus généralement, ils sont capables de diminuer et/ou de retarder le vieillissement cutané, y compris le photovieillissement cutané.

Ces extraits bactériens ont notamment la propriété d'inhiber les lésions du tissu conjonctif cutané consécutives notamment aux expositions UV. Ces extraits bactériens ont en effet la propriété d'inhiber la libération d'élastase dans les zones d'inflammation comme montré dans la partie expérimentale ci-après, ils ont aussi la propriété d'inhiber l'activité d'élastase. Plus généralement, ces extraits bactériens, lorsqu'ils sont appliqués sur la peau, ont des propriétés anti-inflammatoires et apaisantes, et améliorent l'aspect de la peau présentant une inflammation locale ou des micro-inflammations, y compris après l'exposition au soleil.

Ces extraits bactériens ont en outre un effet inhibiteur de l'activité de hyaluronidase. Ainsi, ils permettent de prévenir ou de traiter la sécheresse de la peau, y compris après exposition au soleil et dans les cas de vieillissement cutané naturel ou prématuré, ainsi que dans les cas de photovieillissement. En outre, ils améliorent la tonicité de la peau en favorisant l'hydratation du derme.

L'invention a donc pour objet l'utilisation d'un extrait d'au moins une bactérie de la famille des Pseudomonadacées comme agent cosmétique permettant de lutter contre le vieillissement cutané, naturel ou prématuré, y compris le photovieillissement, d'améliorer l'aspect et la tonicité des peaux sèches et/ou de conserver ou d'améliorer l'élasticité de la peau. Dans la présente demande, l'expression "lutter contre" le vieillissement cutané signifie prévenir ou retarder ou encore traiter, le vieillissement cutané.

Parmi les bactéries utilisables selon l'invention, on peut citer notamment :
- *Pseudomonas vesicularis,* dont l'un des types est la souche déposée à l'ATCC sous le n° 11426.
- *Pseudomonas maltophilia* dont l'un des types est la souche déposée à l'ATCC sous le n° 13637.

*Pseudomotias maltophilia* est encore appelé *Stenotrophomonas maltophilia.*

Dans la présente demande, l'expression "extraits de bactéries" ou "extraits bactériens" désigne aussi bien les biomasses obtenues après culture des bactéries que les produits obtenus à partir de ces biomasses, notamment après purification et/ou stérilisation. Par exemple, les biomasses peuvent être éventuellement au moins partiellement déshydratées et/ou broyées. Elles peuvent être stérilisées, par exemple par chauffage. Dans la présente demande, la notion d'extraits englobe également des dérivés obtenus par modification chimique de certains groupements fonctionnels (amines par exemple).

Le procédé de préparation d'un extrait bactérien utilisé selon l'invention comprend les étapes consistant à cultiver *in vitro* les bactéries selon les méthodes connues, puis à recueillir la biomasse obtenue.

Les bactéries de la famille des Pseudomonadacées sont des bactéries Gram-négatives aérobies strictes. Elles poussent sur les milieux nutritifs ordinaires, par exemple à des températures de l'ordre de 25 à 30°C.

Pour séparer et isoler la biomasse, on peut utiliser diverses méthodes connues telles que la filtration ou la centrifugation. On peut également sécher la biomasse et la concentrer par déshydratation, notamment par chauffage sous pression réduite (par exemple à une température de l'ordre de 80 à 120°C environ) ou encore par lyophilisation.

On peut utiliser les extraits bactériens sous la forme de dérivés, par exemple de dérivés au moins partiellement acylés. On effectue l'acylation à l'aide d'un anhydride d'acide carboxylique ou avec un chlorure d'acide correspondant. On peut utiliser par exemple l'anhydride acétique ou le chlorure d'acétyle. On effectue la réaction d'acylation de façon à ce qu'au moins une partie des groupements amines primaires et secondaires présents dans la biomasse bactérienne soient acylés. On détermine aisément les proportions d'agents d'acylation et les conditions de la réaction d'acylation par dosage selon les méthodes classiques des groupements amines primaires et secondaires avant et après la réaction d'acylation.

Les extraits de Pseudomonadacées ou leurs dérivés, sont introduits comme ingrédients actifs dans des compositions destinées à être appliquées sur la peau et/ou sur le cuir chevelu. Ces compositions présentent une bonne tolérance cutanée.

L'invention concerne donc une composition cosmétique comprenant comme ingrédient actif un extrait d'au moins une bactérie de la famille des *Pseudomonadacées,* en association avec un excipient acceptable en cosmétologie.

Les excipients présents dans la composition de l'invention sont des excipients usuels. Il s'agit d'excipients compatibles avec un usage sur la peau, sur le cuir chevelu et/ou sur les cheveux.

Dans les compositions utilisées selon l'invention, les extraits bactériens sont présents généralement dans une proportion de 0,0005 % à 5 %, par exemple de 0,001 % à 2 %, et en particulier de 0,01 % à 2 % en poids d'extrait sec bactérien, par rapport au poids de la composition.

Ces compositions peuvent contenir l'extrait bactérien sous forme de dispersions (notamment des émulsions) dans un véhicule approprié tel que par exemple l'eau, les solvants organiques, les corps gras y compris les huiles, et leurs mélanges.

Les compositions peuvent se présenter notamment sous la forme de lotions hydro-alcoolique ou oléo-alcooliques, de gels, d'émulsions de consistance liquide, de crèmes, de sticks solides ou de dispersions vésiculaires Ces compositions peuvent être préparées selon les méthodes usuelles. Elles contiennent les ingrédients et véhicules permettant de les présenter notamment sous l'une des formes qui viennent d'être indiquées. Elles peuvent contenir, outre les extraits bactériens, d'autres ingrédients actifs tels que par exemple des substances absorbant l'ultraviolet, des agents hydratants classiques, des agents anti-radicaux libres, des agents antioxydants, des eaux thermales telles que les eaux des sources thermales de La Roche-Posay, des émollients, des agents antioxydants ou encore d'autres ingrédients usuels tels que des agents conservateurs, des parfums, etc. De tels ingrédients, ainsi que leur utilisation, sont connus et ne seront pas décrits davantage ici.

Les eaux thermales éventuellement utilisées dans la composition de l'invention sont notamment des eaux thermales ayant des propriétés cosmétiques bénéfiques pour la peau. Par exemple, les eaux thermales de La Roche-Posay (France), qui sont riches en sélénium, possèdent notamment des propriétés protectrices vis-à-vis des effets délétères des rayonnements UVA sur la peau, et possèdent également des propriétés antioxydants favorisant la survie des fibroblastes exposés aux rayonnements UVB. Les eaux thermales de La Roche-Posay constituent donc un ingrédient actif intéressant notamment dans les produits cosmétiques destinés à être utilisés pendant ou après l'exposition de la peau au soleil.

L'invention a en outre pour objet un procédé de traitement cosmétique pour lutter contre le vieillissement cutané, caractérisé par le fait que l'on applique sur la peau ou le cuir chevelu une composition telle que définie précédemment. Cette composition est appliquée selon les méthodes usuelles.

Les exemples suivants illustrent l'invention. Dans ces exemples, les pourcentages sont des pourcentages en poids.

### EXEMPLES

### EXEMPLE 1 : Culture de Pseudomonas vesicularis et de Pseudomonas maltophilia

La souche de *Pseudomonas vesicularis* cultivée a été obtenue auprès de l'ATCC (ATCC 11426).

La souche de *Pseudomonas maltophilia* est la souche ATTC 13637.

Les bactéries sont cultivées dans le milieu de culture Nutrient Broth Difco 003 (Medium 3 ATCC). Le pH du milieu est ajusté à 7,15 avant stérilisation à 121 °C pendant au moins 20 minutes.

La culture est effectuée à 26°C sous agitation (100 rpm) en assurant un taux d'oxygène dissous au moins egal à 15%

Après 24 heures de culture, la biomasse est récoltée par centrifugation.

La biomasse peut être stabilisée par chauffage à l'autoclave, lyophilisée, congelée et/ou broyée.

On peut aussi, si désiré, acétyler les groupements amine primaire et secondaire, totalement ou partiellement, par l'action de l'anhydride acétique.

### EXEMPLE 2 : Crème

Cette crème répond à la composition suivante :
- Lyophilisat à base de *Ps*. *vesicularis* obtenu selon l'exemple 1 0,05%
- Carbomer 940* 0,30%
- Triéthanolamine 0,30%
- Acide stéarique 3,00%
- Alcool cétylique 2,00%
- Monostéarate de glycérol autoémulsionnable 3,00%
- Huile de soja 10,00%
- Alcool de lanoline 2,00%
- Myristate d'isopropyle 4,00%
- 2-éthylhexanoate de cétyle et de stéaryle 4,00 %
- Perhydrosqualène 3,00%
- Paraffine 2,00%
- Glycérine 3,00%
- Conservateurs 0,30 %
- Eau thermale de La Roche-Posay** 15,00 %
- Eau purifiée, q.s.p 100,00%

* Carbomer 940 : marque de commerce désignant un acide polyacrylique réticulé
** Centre thermal de La Roche-Posay (France)

On peut remplacer le lyophilisat à base de *Pseudomonas vesicularis* par un lyophilisat à base de *Pseudomonas maltophilia.*

De façon analogue, on a préparé une crème contenant 0,01 % de flyophilisat de *Pseudomonas vesicularis* et 0,05 % de lyophilisat de *Pseudomonas maltophilia.*

Pour préparer cette crème, on chauffe la phase aqueuse contenant la glycérine, les conservateurs et l'eau à 80°C ; on y disperse le Carbomer 940 qui est ensuite neutralisé par la triéthanolamine. La phase grasse, chauffée et homogénéisée à 80°C, est introduite sous vive agitation dans la phase aqueuse. Le lyophilisat de l'exemple 1 est dispersé dans 10 g d'eau et introduit à 40°C dans la crème, sous agitation. L'ensemble est refroidi jusqu'à température ambiante.

Cette crème est appliquée sur la peau du visage et du cou une à deux fois par jour. Elle améliore l'aspect des peaux sèches. Elle permet également d'améliorer la tonicité de la peau.

### EXEMPLE 3 : Lait pour la peau

Ce lait a la composition suivante :
- Lyophilisat de *Ps. vesicularis* obtenu selon l'exemple 1 0,10 %
- Monostéarate de glycérol autoémulsionnable 3,00 %
- Vaseline 1,50%
- Huile de vaseline 2,50 %
- Huile de son de riz 1,50 %
- Huile de silicone volatile 5,00%
- Beurre de karité 3,00%
- Carbomer 940 0,20 %
- Triéthanolamine 0,20%
- Gomme de xanthane 0,10%
- Glycérine 3,00%
- Parfum 0,10%
- Conservateurs 0,30%
- Eau q.s.p 100,00%

Ce lait est préparé d'une façon analogue à celle décrite à l'exemple 2.

Appliqué sur la peau après une exposition au soleil, il possède des propriétés apaisantes.

Appliqué sur la peau du visage, ce lait diminue l'effet de vieillissement cutané accéléré observé notamment chez les personnes s'exposant de façon répétée au soleil.

### EXEMPLE 4 : Crème

On a préparé selon le même mode opératoire qu'à l'exemple 2 une émulsion ayant la composition suivante :
- Lyophilisat de *Ps. vesicularis* obtenu selon l'exemple 1 0,10%
- Base auto-émulsionnable 20,00%
- Huile de vaseline codex 5,00%
- Glycérine 5,00%
- Stéarate d'aluminium .0,50%
- EDTA di-potassique 0,05%
- Sulfate de magnèsium 0,70%
- Agents conservateurs 0,20%
- Antioxydants 0,05%
- Parfum 0,30%
- Eau q.s.p 100,00%

Dans la formulation ci-dessus, on peut remplacer le lyophilisat de *Pseudomonas vesicularis* par un lyophilisat de *Pseudomonas maltophilia.* On peut également utiliser un mélange des deux lyophilisats.

La base auto-émulsionnable comprend :
- Huile minérale
- Vaseline codex
- Ozokérite
- Oléate de glycérol
- Lanoline liquide

Cette crème, appliquée sur la peau, permet de réduire les effets du vieillissement cutané et/ou du photovieillissement cutané. Elle permet également d'améliorer le degré d'hydratation de la peau de sujets âgés.

### EXEMPLE 5 : Emulsion anti-solaire

Cette émulsion permet de protéger la peau contre des rayons ultraviolets. Elle répond à la formule suivante :
- Lyophilisat de l'exemple 1 1,00%
- Acide stéarique 3,00%
- Alcool cétylique 1,50%
- Monostéarate de glycérol auto-émulsionnable 3,00%
- Huile de tournesol 8,00%
- Polyacrylamide 3,00%
- Méthoxycinnamate d'octyle 4,00%
- Sel de triéthanolamine de l'acide benzène-1,4-di-(3-méthylidène)-10-campho sulfonique (Mexoryl SX) 2,60%
- Glycérol 5,00%
- Tocophérol 2,00%
- Conservateurs 0,30%
- Ethylènediaminetétraméthylène phosphonate (sel pentasodique) 0,10 %
- Eau purifiée, q.s.p. 100,00%

### EXEMPLE 6 : Test d'inhibition de l'élastase

Le test est effectué à partir d'élastase isolée de leucocytes humains.

Le test est réalisé selon la méthode décrite par ADEYEMI E.O. et al., J. Pharm Pharmacol., 42:487-490 (1990). Les essais sont effectués avec un lyophilisat obtenu comme décrit à l'exemple 1.

Le lyophilisat provenant de la culture de *Pseudomonas maltophilia,* à une concentration de 0,05 g/l, diminue l'activité de l'élastase de 36 %. A une concentration de 0,1 g/l, le lyophilisat provenant de la culture de *Pseudomonas vesicularis* diminue l'activité de l'élastase de 33 %, et le lyophilisat en provenance d'une culture de *Pseudomonas maltophilia* diminue l'activité de l'élastase de 53 %.

### EXEMPLE 7 : Effet inhibiteur de l'activité de hyaluronidase

Le test est réalisé selon la méthode classique décrite dans Worthington Enzyme Manual, Enzymes and related biochemicals, Worthington Biochemical Corps., Frehold, New Jersey 07728, USA (1993).

Le lyophilisat bactérien étudié est un lyophilisat de *Pseudomonas vesicularis* Il est mis en solution en tampon phosphate 0,1 M, pH 5,3.

Les réactifs utilisés sont l'acide hyaluronique (Sigma H-1876) et la hyaluronidase Sigma type IV-S (H-3884). On mélange l'acide hyaluronique et la hyaluronidase en tampon phosphate de façon à obtenir une solution contenant 0,6 g/l d'acide hyaluronique et 0,25 g/l de hyaluronidase.

On laisse incuber pendant 15 minutes à 37°C.

On ajoute alors une solution d'albumine bovine à 1 % dans un tampon acétate 0,5 M, pH 4,2, pour précipiter l'acide hyaluronique.

On mesure alors la quantité d'acide hyaluronique non dégradé par mesure de l'absorption de la lumière à une longueur d'onde de 540 nm.

Le lyophilisat de *Pseudomonas vesicularis,* à une concentration de 0,1%, inhibe de 30 % l'activité de la hyaluronidase.

## Revendications

1. Utilisation d'un extrait d'au moins une bactérie de la famille des pseudomonadacées comme agent cosmétique destiné à lutter contre le vieillissement cutané y compris le photovieillissement, à améliorer l'aspect et la tonicité des peaux sèches, et/ou à conserver ou à améliorer l'étasticité de la peau, ledit extrait étant constitué soit par la biomasse obtenue après culture de ladite bactérie, suivie éventuellement d'une purification, d'un broyage, d'une déshydratation au moins partielle et/ou d'une stérilisation, soit par un dérivé, obtenu par modification chimique de certains groupements fonctionnels, de ladite biomasse, ledit dérivé possédant des propriétés d'inhibition de l'activité d'élastase et/ou d'inhibition de l'activité de hyaluronidase.

2. Utilisation selon la revendication 1, dans laquelle ladite bactérie appartient au genre *Pseudomonas*.

3. Utilisation selon la revendication 2, dans laquelle ladite bactérie est choisie parmi *Pseudomonas* v*esicularis* et *Pseudomonas maltophilia*.

4. Utilisation selon l'une quelconque des revendications précédentes, dans laquelle ledit extrait est constitué par la biomasse bactérienne obtenue après culture de la bactérie, ladite biomasse étant éventuellement broyée et/ou au moins partiellement déshydratée.

5. Utilisation selon l'une quelconque des revendications précédentes, dans laquelle ledit extrait est appliqué sous la forme d'une composition contenant une proportion de 0,0005 % à 5 % en poids d'extrait sec bactérien par rapport au poids total de la composition.

6. Utilisation selon la revendication précédente, dans laquelle ladite proportion est dans la gamme de 0,001 % à 2 % en poids.

7. Procédé de traitement cosmétique destiné à lutter contre le vieillissement cutané, y compris le photovieillissement, à améliorer l'aspect et la tonicité des peaux sèches, et/ou à conserver ou améliorer l'élasticité de la peau, ledit procédé comprenant l'étape consistant à appliquer sur la peau ou sur le cuir chevelu un extrait d'au moins une bactérie de la famille des Pseudomonadacées, ledit extrait étant constitué soit par la biomasse obtenue après culture de ladite bactérie, suivie éventuellement d'une purification, d'un broyage, d'une déshydratation au moins partielle et/ou d'une stérilisation, soit par un dérivé, obtenu par modification chimique de certains groupements fonctionnels, de ladite biomasse, ledit dérivé possédant, des propriétés d'inhibition de l'activité d'élastase et/ou d'inhibition de l'activité de hyaluronidase.

8. Procédé selon la revendication précédente, présentant l'une au moins des caractéristiques suivantes :
- la bactérie appartient aux genres *Pseudomonas* ;
- la bactérie appartient à l'espèce *Pseudomonas vesicularis* où *Pseudamonas maltophilia* ;
- ledit extrait est constitué par la biomasse bactérienne obtenue après culture de la bactérie, ladite biomasse étant éventuellement broyée et/ou, au moins partiellement déshydratée ;
- on applique ledit extrait sous la forma d'une composition contenant une proportion de 0,0005 % à 5 % et en particulier de 0,001 à 2 % en poids d'extrait sec bactérien par rapport an poids total de la composition.

9. Procédé selon l'une quelconque des revendications 7 et 8, destiné à lutter contre le photovieillissement cutané.

10. Procédé selon l'une quelconque des revendications 7 et 8, destiné à améliorer l'aspect des peaux sèches.

11. Procédé selon l'une quelconque des revendications 7 et 8, destiné à améliorer la tonicité des peaux sèches.

12. Procédé selon l'une quelconque des revendications 7 et 8, destiné à conserver ou à améliorer l'élasticité de la peau.

13. Utilisation d'un extrait d'au moins une bactérie de la famille des *Pseudomonadacées* comme ingrédient actif dans la fabrication d'une composition destinée à améliorer l'aspect des peaux présentant une réaction inflammatoire locale, ledit extrait étant constitué soit par la biomasse obtenue après culture de ladite bactérie, suivie éventuellement d'une purification, d'un broyage, d'une déshydratation au moins partielle et/ou d'une stérilisation, soit par un dérivé, obtenu par modification chimique de certains groupements fonctionnels, de ladite biomasse, ledit dérivé possédant des propriétés d'inhibition de l'activité d'élastase et/ou d'inhibition de l'activité de hyaluronidase.

14. Utilisation selon la revendication 13, **caractérisée en ce qu'**elle présente l'une des caractéristiques suivantes :
- la bactérie appartient aux genres *Pseudomonas* ;
- la bactérie appartient à l'espèce *Pseudomonas vesicularis* ou *Pseudomonas maltophilia* ;
- ledit extrait est constitué par la biomasse bactérienne obtenue après culture de la bactérie, ladite biomasse étant éventuellement broyée et/ou au moins partiellement déshydratée ;
- ledit extrait est présent dans la composition en une proportion de 0,0005 % à 5 % et en particulier de 0,001 à 2 % en poids d'extrait sec bactérien par rapport au poids total de la composition.

15. Composition cosmétique comprenant comme ingrédient actif un extrait d'au moins une bactérie de la famille des *Pseudomonadacées,* en association avec un excipient acceptable en cosmétologie, ledit extrait étant constitué soit par la biomasse obtenue après culture de ladite bactérie, suivie éventuellement d'une purification, d'un broyage, d'une déshydratation au moins partielle et/ou d'une stérilisation, soit par un dérivé, obtenu par modification chimique de certains groupements fonctionnels, de ladite biomasse, ledit dérivé possédant des propriétés d'inhibition de l'activité d'élastase et/ou d'inhibition de l'activité de hyaluronidase.

16. Composition selon la revendication 15, présentant l'une au moins des caractéristiques suivantes :
- la bactérie appartient au genre *Pseudomonas ;*
- la bactérie appartient à l'espèce *Pseudomonas vesicularis* ou *Pseudomonas maltophilla* ;
- ledit extrait est constitué par la biomasse Bactérienne obtenue après culture de la bactérie, ladite biomasse étant au moins partiellement déshydratée ;
- ladite composition contient une proportion de 0,0005 % à 5% et en particulier de 0,001 % à 2 % en poids d'extrait sec bactérien par rapport au poids total de la composition.

## Patentansprüche

1. Verwendung eines Extrakts aus mindestens einer Bakterie aus der Familie der Pseudomonadazeen als kosmetisches Mittel, vorgesehen zur Bekämpfung der Hautalterung einschließlich der Fotoalterung, zur Verbesserung der Erscheinung und des Tonus' trockener Haut und/oder zur Konservierung oder zur Verbesserung der Elastizität der Haut, wobei der Extrakt entweder aus der Biomasse, erhalten nach Kultur der Bakterie, eventuell gefolgt von einer Reinigung, einem Malschritt, einer zumindest partiellen Dehydratisierung und/oder einer Sterilisation, oder aus einem Derivat besteht, das durch die chemische Modifikation bestimmter funktioneller Gruppen der Biomasse erhalten wird, wobei das Derivat die Eigenschaft besitzt, die Elastase-Aktivität zu inhibieren und/oder die Hyaluronidase-Aktivität zu inhibieren.

2. Verwendung nach Anspruch 1, in der die Bakterie der Gattung *Pseudomonas* angehört.

3. Verwendung nach Anspruch 2, in der die Bakterie ausgewählt ist aus *Pseudomonas vesicularis* und *Pseudomonas maltophilia.*

4. Verwendung nach einem der vorhergehenden Ansprüche, bei der der Extrakt aus der bakteriellen Biomasse besteht, die nach der Kultur der Bakterie erhalten wird, wobei die Biomasse eventuell gemahlen ist und/oder zumindest partiell dehydratisiert ist.

5. Verwendung nach einem der vorhergehenden Ansprüche, in der der Extrakt in Form einer Zusammensetzung angewandt wird, enthaltend einen Anteil von 0,0005 % bis 5 Gew.-% an trockenem Bakterienextrakt, bezogen auf das Gesamtgewicht der Zusammensetzung.

6. Verwendung nach dem vorhergehenden Anspruch, in der der genannte Anteil im Bereich von 0,001 % bis 2 Gew.-% ist.

7. Kosmetisches Behandlungsverfahren, bestimmt zur Bekämpfung der Hautalterung, einschließlich der Fotoalterung, zur Verbesserung der Erscheinung und des Tonus' trockener Haut und/oder zur Konservierung oder Verbesserung der Elastizität der Haut, wobei das Verfahren den Schritt umfasst, bestehend aus dem Anwenden eines Extrakts aus mindestens einer Bakterie der Familie der Pseudomonadazeen auf der Haut oder der Kopfhaut, wobei der Extrakt entweder aus der Biomasse, die nach der Kultur der Bakterie erhalten worden ist, gefolgt eventuell von einer Reinigung, einem Malschritt, einer zumindest partiellen Dehydratisierung und/oder einer Sterilisation, oder aus einem Derivat besteht, das durch chemische Modifikation bestimmter funktioneller Gruppen der besagten Biomasse erhalten wird, wobei das Derivat die Eigenschaft besitzt, die Elastase-Aktivität zu inhibieren und/oder die Hyaluronidase-Aktivität zu inhibieren.

8. Verfahren nach dem vorhergehenden Anspruch, das mindestens eines der folgenden Charakteristika aufweist:
• die Bakterie gehört der Gattung *Pseudomonas* an;
• die Bakterie gehört der Art *Pseudomonas vesicularis* oder *Pseudomonas maltophilia* an;
• der Extrakt besteht aus der bakteriellen Biomasse, die nach der Kultur der Bakterie erhalten wird, wobei die Biomasse eventuell gemahlen und/oder wenigstens teilweise dehydratisiert ist;
• man wendet den Extrakt in Form einer Zusammensetzung an, die einen Anteil von 0,0005 % bis 5 % und insbesondere von 0,001 bis 2 Gew.-% an trockenem Bakterienextrakt, bezogen auf das Gesamtgewicht der Zusammensetzung, enthält.

9. Verfahren nach einem der Ansprüche 7 und 8, vorgesehen zur Bekämpfung der Fotohautalterung.

10. Verfahren nach einem der Ansprüche 7 und 8, vorgesehen zur Verbesserung der Erscheinung trockener Haut.

11. Verfahren nach einem der Ansprüche 7 und 8, vorgesehen zur Verbesserung des Tonus' trockener Haut.

12. Verfahren nach einem der Ansprüche 7 und 8, vorgesehen zur Konservierung oder zur Verbesserung der Elastizität der Haut.

13. Verwendung eines Extrakts mindestens einer Bakterie der Familie der *Pseudomonadazeen* als aktiver Inhaltsstoff bei der Herstellung einer Zusammensetzung, vorgesehen zur Verbesserung des Erscheinungsbildes von Haut, die eine lokale entzündliche Reaktion aufweist, wobei der Extrakt entweder aus der Biomasse, die nach der Kultur der Bakterie erhalten wird, eventuell gefolgt von einer Reinigung, einem Malschritt, einer zumindest partiellen Dehydratisierung und/oder einer Sterilisation, oder aus einem Derivat besteht, das durch chemische Modifikationen bestimmter funktioneller Gruppen der Biomasse erhalten worden ist, wobei das Derivat die Eigenschaft besitzt, die Elastase-Aktivität zu inhibieren und/oder die Hyaluronidase-Aktivität zu inhibieren.

14. Verwendung nach Anspruch 13, **dadurch** charakteristiert, dass sie eine der folgenden Charakteristika aufweist:
• die Bakterie gehört der Gattung *Pseudomonas* an;
• die Bakterie gehört der Art *Pseudomonas vesicularis* oder *Pseudomonas maltophilia* an;
• der Extrakt besteht aus der bakteriellen Biomasse, die nach der Kultur der Bakterie erhalten wird, wobei die Biomasse eventuell gemahlen ist und/oder zumindestens partiell dehydratisiert ist;
• der Extrakt liegt in der Zusammensetzung in einem Anteil von 0,0005 % bis 5 % und insbesondere von 0,001 bis 2 Gew.-% an trockenem Bakterienextrakt, bezogen auf das Gesamtgewicht der Zusammensetzung, vor.

15. Kosmetische Zusammensetzung, umfassend als aktiven Inhaltsstoff einen Extrakt mindestens einer Bakterie der Familie der *Pseudomonadazeen* in Verbindung mit einem kosmetisch annehmbaren Hilfsstoff, wobei der Extrakt entweder aus der Biomasse, die nach der Kultur der Bakterie erhalten wird, eventuell gefolgt von einer Reinigung, einem Malschritt, einer zumindest partiellen Dehydratisierung und/oder einer Sterilisation, oder aus einem Derivat besteht, erhalten durch chemische Modifikation bestimmter funktioneller Gruppen der Biomasse, wobei das Derivat die Eigenschaft besitzt, die Elastase-Aktivität zu inhibieren und/oder die Hyaluronidase-Aktivität zu inhibieren.

16. Zusammensetzung nach Anspruch 15, die mindestens eine der folgenden Charakteristika aufweist:
• die Bakterie gehört der Gattung *Pseudomonas* an;
• die Bakterie gehört der Art *Pseudomonas vesicularis* oder *Pseudomonas maltophilia* an;
• der Extrakt besteht aus der bakteriellen Biomasse, die nach der Kultur der Bakterie • erhalten wird, wobei die Biomasse zumindestens partiell dehydratisiert ist;
• die Zusammensetzung enthält einen Anteil von 0,0005 % bis 5 % und insbesondere von 0,001 % bis 2 Gewichts-% an bakteriellem Trockenextrakt, bezogen auf das Gesamtgewicht der Zusammensetzung.

## Claims

1. Use of an extract of at least one bacterium from the family Pseudomonadaceae, as a cosmetic agent intended to combat ageing of the skin, including photoageing, to improve the appearance and tonicity of dry skin and/or to preserve or improve skin elasticity, said extract consisting either of the biomass obtained after culturing said bacterium, optionally followed by purification, grinding, at least partial dehydration and/or sterilization, or of a derivative, obtained by chemical modification of certain functional groups, of said biomass, said derivative having elastase activity inhibition and/or hyaluronidase activity inhibition properties.

2. Use according to Claim 1, in which said bacterium belongs to the genus *Pseudomonas.*

3. Use according to Claim 2, in which said bacterium is chosen from *Pseudomonas vesicularis* and *Pseudomonas maltophilia.*

4. Use according to any one of the preceding claims, in which said extract consists of the bacterial biomass obtained after culturing the bacterium, said biomass optionally being ground and/or at least partially dehydrated.

5. Use according to any one of the preceding claims, in which said extract is applied in the form of a composition containing a proportion of 0.0005% to 5% by weight of bacterial solids relative to the total weight of the composition.

6. Use according to the preceding claim, in which said proportion is within the range of 0.001% to 2% by weight.

7. Cosmetic treatment method intended to combat ageing of the skin, including photoageing, to improve the appearance and tonicity of dry skin and/or to preserve.or improve skin elasticity said method comprising the step consisting in applying an extract of at least one bacterium from the family Pseudomonadaceae to the skin or to the scalp, said extract either consisting of the biomass obtained after culturing said bacterium; optionally followed by purification, grinding, at least partial dehydration and/or sterilization, or of a derivative, obtained by chemical modification of certain functional groups, of said biomass, said derivative having elastase activity inhibition and/or hyaluronidase activity inhibition properties.

8. Method according to the preceding claim, having at least one of the following characteristics:
- the bacterium belongs to the genus *Pseudomonas;*
- the bacterium belongs to the species *Pseudomonas vesicularis* or *Pseudomonas maltophilia;*
- said extract consists of the bacterial biomass obtained after culturing the bacterium, said biomass optionally being ground and/or at least partially dehydrated;
- said extract is applied in the form of a composition containing a proportion of 0.0005% to 5%, and in particular of 0.001 to 2%, by weight of bacterial solids relative to the total weight of the composition.

9. Method according to either of Claims 7 and 8, intended to combat photoageing of the skin.

10. Method according to either of Claims 7 and 8, intended to improve the appearance of dry skin.

11. Method according to either of Claims 7 and 8, intended to improve the tonicity of dry skin.

12. Method according to either of Claims 7 and 8, intended to preserve or improve skin elasticity.

13. Use of an extract of at least one bacterium from the family *Pseudomonadaceae,* as active ingredient to manufacture a composition intended to improve the appearance of skin which exhibits a local inflammatory reaction, said extract consisting either of the biomass obtained after culturing said bacterium, optionally followed by purification, grinding, at least partial dehydration and/or sterilization, or of a derivative, obtained by chemical modification of certain functional groups, of said biomass, said derivative having elastase activity inhibition and/or hyaluronidase activity inhibition properties.

14. Use according to Claim 13, **characterized in that** it presents one of the following characteristics:
- the bacterium belongs to the genus *Pseudomonas;*
- the bacterium belongs to the species *Pseudomonas vesicularis* or *Pseudomonas maltophilia;*
- said extract consists of the bacterial biomass obtained after culturing the bacterium, said biomass being optionally being ground and/or at least partially dehydrated;
- said extract being present in the composition in a proportion of 0.0005% to 5%, and in particular of 0.001% to 2%, by weight of bacterial solids relative to the total weight of the composition.

15. Cosmetic composition comprising, as an active ingredient, an extract of at least one bacterium from the family *Pseudomonadaceae,* in combination with an excipient which is acceptable in cosmetology, said extract consisting either of the biomass obtained after culturing said bacterium, optionally followed by purification, grinding, at least partial dehydration and/or sterilization, of a derivative, obtained by chemical modification of certain functional groups; of said biomass, said derivative having elastase activity inhibition and/or hyaluronidase activity inhibition properties.

16. Composition according to Claim 15, having at least one of the following characteristics:
- the bacterium belongs to the genus *Pseudomonas;*
- the bacterium belongs to the species *Pseudomonas vesicularis* or *Pseudomonas maltophilia;*
- said extract consists of the bacterial biomass obtained after culturing the bacterium, said biomass being at least partially dehydrated;
- said composition contains a proportion of 0.0005% to 5%, and in particular of 0.001% to 2%, by weight of bacterial solids relative to the total weight of the composition.
